# EUROPEAN PATENT SPECIFICATION

(11) **EP 1 302 209 B1**
(45) Date of publication and mention of the grant of the patent: **09.03.2005**
(21) Application number: 02380206.9
(22) Date of filing: 04.10.2002
(51) Int. Cl.: A61L 27/40, A61L 29/12, A61L 31/12

(54) **Composite laminar material for medical and surgical applications**
Mehrschichtiges, medizinisches Material für die Chirurgie
Article médical stratifié à usage chirurgical

(30) Priority: 10.10.2001 ES 200102472 U
(43) Date of publication of application: 16.04.2003
(73) Proprietor: Thermical Medic, S.L., 08960 Sant Just Desvern (ES)
(72) Inventor: Martinez Punet, Jaime, 08860 Castelldefels (ES)
(74) Representative: SUGRANES - VERDONCES - FERREGÜELA

(56) References cited:
- EP-A- 1 084 823
- WO-A-87/05563
- DE-A- 3 113 888
- US-A- 4 360 984

## Description

### Technical sector of the invention

The present invention relates to a composite laminar material for applications requiring a coating with physically resistant and protective properties both for people or things, particularly for medical or surgical applications.

### Background to the invention

Whether for the manufacture of clinical articles or the care of patients in surgery, numerous embodiments of composite materials are known, which consist of at least one polymeric layer in combination with others that give mechanical resistance to the items, with properties which thermally or electrically insulate and which have the possibility of colouring.

The materials of this type also cover another wide range of applications, for example in the field of medical-surgical accessories, in the field of foodstuff preservation, for the protection of other materials and for the manufacture and development of items of maximum safety for their specific ability as insulators from both heat and electricity.

Spanish patent ES-P9901942 describes a laminar composite material for this type of application, in particular a medical-surgical one, composed of successive layers of foam, metalled polyester, polythene and an outer nylon® material which, even though it provides an acceptable functionality, it is not completely free of problems. Among these problems it is worth pointing out that the nylon can make the product inflexible and thus, less easy to use. Furthermore, this composite material can work out expensive because of the presence of the nylon and due to the manufacturing process itself.

The aim of the present invention is to disclose a new composite laminar material of this type which is free of the previously mentioned problems.

### Explanation of the invention

To such purpose, the object of the present invention is a composite laminar material for medical and surgical applications, novel in structure, which is essentially characterised in that it consists of a first outer polyurethane foam layer, a second intermediate layer of metalled polyester and a final outer finishing layer, selected this outer finishing layer from a transparent polyester film or a coloured ink plus a protective varnish.

According to another embodiment, said final finishing layer is a protective varnish which enables the fixing of an outer coloured ink easier.

### Brief description of the drawings

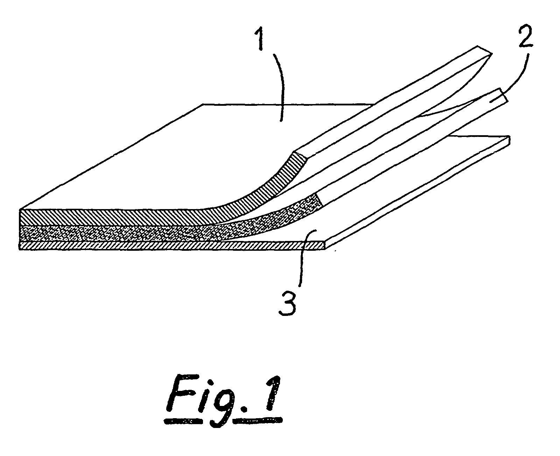

Below is a description of a preferred, though not exclusive, form of embodiment of the laminar material of the present invention, which is attached with a sheet of drawings to aid comprehension, given merely by way of non-limiting example, wherein in Fig. 1 a diagrammatic perspective view of a rectangular portion of the laminar material is represented according to the invention, with the different layers partially detached in order to aid comprehension.

The new metallic material that we are discussing herein, which is of particular use for medical and surgical applications, consists of the superimposition of three films of different components in layer form, according to the following configuration: a first outer polyurethane foam layer 1, a second intermediate metalled polyester layer 2 and a final outer finishing layer 3. The final finishing layer is selected from a transparent polyester film, a colour ink plus a protective varnish, or a protective varnish which eases for the fixing of an outer coloured ink.

This new material now being applied for as the object of the present invention has a great capacity for clinical use, both for the manufacture of medical-surgical materials or for clinical application and in particular for the care of patients undergoing surgery, being especially useful for surgery itself, in view of the fact that they have flexible properties, are insulators from both heat and electricity, and have anti-dazzle properties and colouring possibilities.

Furthermore, it should be borne in mind that the material herein presented is not only good for the protection and manufacture of objects, or items for the application indicated, such as material, but can also be used for the covering of parts or for the protection of other materials.

The combination of the three superimposed films 1, 2 and 3 which make up this new laminar material result in a new material, which has the following principal characteristics:
- It is flexible. Due to the composition of the layers and absence of inflexible materials, such as nylon, it can be folded and bent easily without any risk of breaking or unwanted deformations, which is particularly suitable for applications such as dressings and medical-surgical applications in general.
- It is dielectric. Therefore, it should be said that the electrical conductivity of the same is almost nonexistent and its application is thus valid in the protection of the hospitalised, and particularly offers a great deal of safety in the handling of surgical appliances that work on electricity.
- It is isothermal. Also springing from this is its great applicability in the clothing industry or use as a clinical blanket or similar.
- It is waterproof, due to the components of same and is absorbent at the same time.
- It is aseptic. That is, it repels the spread of infections, particularly in the clinical field, because of the fact that its high waterproof capability does not facilitate transpiration.

It should also be mentioned that this novel embodiment of laminar material according to the invention presents other properties of lesser importance, such as its softness, its quietness when handled, its lack of transpiration for alcoholic products and detergents, so it is an ideal blanket and dressing, and it does not stain. It is also resistant to the physical deterioration of the metallization, due to its film finish. which is protective, so it is practically impossible to damage or break this metallization.

Having sufficiently described the nature of the new material object of the present invention, it is herein stated that anything which does not vary or alter its principle may be subject to variations in detail and/or replaced by technical equivalent, the most important being noted in the following claims.

## Claims

1. Composite laminar material for medical and surgical applications, **characterised in that** it consists of a first outer polyurethane foam layer (1), a second intermediate metallic polyester layer (2) and a final outer finishing layer(3) selected from a transparent polyester film or a colour ink plus a protective varnish.

2. Composite material according to claim 1**characterised in that** the final finishing layer (3) is a protective varnish which enables the fixing of an outer coloured ink.

## Patentansprüche

1. Verbundlaminatmaterial für medizinische und chirurgische Anwendungen, **dadurch gekennzeichnet, daß** es aus einer ersten äußeren Polyurethanschaumstoffschicht (1), einer zweiten zwischenliegenden metallischen Polyesterschicht (2) und einer abschließenden äußeren Veredelungsschicht (3) besteht, die ausgewählt ist aus einer transparenten Polyesterfolie oder einer farbigen Durckfarbe plus einem Schutzlack.

2. Verbundmaterial nach Anspruch 1, **dadurch gekennzeichnet, daß** die abschließende Veredelungsschicht (3) ein Schutzlack ist, der die Befestigung einer äußeren farbigen Druckfarbe ermöglicht.

## Revendications

1. Matériau stratifié composite pour applications médicales et chirurgicales, **caractérisé en ce qu'**il se compose d'une première couche de mousse de polyuréthane externe (1), d'une deuxième couche de polyester métallique intermédiaire (2) et d'une couche de finition externe finale (3) consistant en un film de polyester transparent ou une encre colorée additionnée d'un vernis protecteur.

2. Matériau composite selon la revendication 1, **caractérisé en ce que** la couche de finition finale (3) est un vernis protecteur qui permet le fixage d'une encre colorée à l'extérieur.
